# FASCICULE DE BREVET EUROPEEN

(11) **EP 1 196 365 B1**
(45) Date de publication et mention de la délivrance du brevet: **03.03.2004**
(21) Numéro de dépôt: 00915235.6
(22) Date de dépôt: 29.03.2000
(51) Int. Cl.: C07C 43/13, C07C 43/178

(54) **DIOL FLUORE ET SON PROCEDE DE PREPARATION**
FLUORINIERTE DIOLE UND VERFAHREN ZU IHRER HERSTELLUNG
FLUORINATED DIOL AND METHOD FOR THE PRODUCTION THEREOF

(30) Priorité: 01.04.1999 FR 9904093
(43) Date de publication de la demande: 17.04.2002
(73) Titulaire: E.I. du Pont de Nemours and Company, Wilmington, DE 19898 (US)
(72) Inventeur: LINA, Marie-José, F-69005 Lyon (FR); LACROIX, Eric, F-69480 Ambérieux d'Azergues (FR); VANPOULLE, Sophie, F-91190 Gif sur Yvette (FR); ORCEL, Gérard, F-78600 Maison Laffite (FR); OVERTON, Robert, Lenoir, NC 28645 (US)
(74) Mandataire: Abitz, Walter, Dr.-Ing.
(86) Numéro de dépôt international: PCT/FR2000/000778
(87) Numéro de publication internationale: WO 2000/059856

(56) Documents cités:
- EP-A- 0 565 425
- DE-A- 2 336 913
- FR-A- 2 712 291

## Description

L'invention a pour objet un diol fluoré, utile notamment pour la préparation d'un matériau polymère de type polyuréthanne pour revêtement de fibre optique, ainsi que son procédé de préparation.

On sait que les fibres optiques comportent un double revêtement polymère constitué d'un revêtement primaire plastifié en contact avec la fibre de verre et surmonté d'un revêtement secondaire. Ce double revêtement protège la fibre des agressions mécaniques ou chimiques susceptibles de provoquer des défauts d'atténuation pour les transmissions optiques.

Chaque revêtement doit posséder une bonne adhérence sur le support qui lui est destiné, et ses propriétés physiques doivent être compatibles avec les conditions de fibrage, en particulier la vitesse de fibrage et l'utilisation finale de la fibre. Le revêtement primaire doit absorber les microcourbures et les contraintes éventuelles sur le verre. Le revêtement secondaire confère à la fibre ses propriétés mécaniques.

A l'heure actuelle les revêtements primaires et secondaires sont des revêtements du type polyuréthanne acrylate photoréticulés sous rayonnement U.V..

La demande EP-A-0565425 décrit un matériau polymère de type polyuréthanne acrylate fluoré pour revêtement de fibre optique, matériau à base d'au moins un diol, un diisocyanate et un acrylate, caractérisé en ce que au moins un des composés précédents contient du fluor et que au moins un des composés précédents contient du soufre.

Ce matériau présente de bonnes caractéristiques mécaniques, notamment une résistance à la fatigue en mode statique améliorée. Cependant, il fait par exemple emploi d'un diol soufré, ce qui implique des coûts de production élevés, du fait de l'étape intermédiaire de préparation du thiol qui conduit à des sous-produits qu'il faut éliminer. Le problème est identique avec les autres composés soufrés utilisés pour la préparation du matériau objet de la demande EP-A-0565425.

On recherche donc un matériau présentant les mêmes propriétés mécaniques, mais qui ne fasse pas appel à des produits soufrés, notamment qui ne fasse pas appel à des diols soufrés.

On recherche donc en particulier un diol fluoré non soufré.

Ainsi, l'invention fournit un diol fluoré répondant à la formule (I):

CₙF₂ₙ₊₁―A―CH₂OCH₂―C(CH₂OH)₂―R

dans laquelle n vaut de 2 à 20 et A signifie ―CH=CH― ou ―CH₂CH₂― et R est un groupe alkyle contenant 1 à 4 atomes de carbone.

Selon un mode de réalisation, le diol fluoré est insaturé et correspond à la formule:

CₙF₂ₙ₊₁―CH=CH―CH₂OCH₂―C(CH₂OH)₂―R.

Selon un autre mode de réalisation, le diol fluoré est saturé et correspond à la formule:

CₙF₂ₙ₊₁―CH₂CH₂―CH₂OCH₂―C(CH₂OH)₂―R.

Selon un mode de réalisation, dans la formule I, R est C₂H₅.

Selon un mode de réalisation, dans la formule (I), n est un entier et est compris entre 6 et 14, inclusivement.

Selon un mode de réalisation, dans la formule (I), CₙF₂ₙ₊₁ résulte d'un mélange et n est compris entre 6 et 14, inclusivement.

Selon un mode de réalisation, dans la formule (I), n est compris entre 6 et 8, inclusivement.

L'invention fournit aussi un procédé de préparation d'un diol fluoré selon l'invention, lorsque celui-ci est insaturé, comprenant la réaction radicalaire de CₙF₂ₙ₊₁I sur le monoallyl éther de triméthylol alcane, l'alcane correspondant au groupe R augmenté d'un atome de carbone, puis la déshydroiodation.

L'invention fournit aussi un procédé de préparation d'un diol fluoré selon l'invention, lorsque celui-ci est saturé, comprenant la réaction radicalaire de CₙF₂ₙ₊₁I sur le monoallyl éther de triméthylol alcane, l'alcane correspondant au groupe R augmenté d'un atome de carbone, puis la réduction directe.

L'invention fournit aussi un procédé de préparation d'un diol fluoré selon l'invention, lorsque celui-ci est saturé, comprenant la réaction radicalaire de CₙF₂ₙ₊₁I sur le monoallyl éther de triméthylol alcane, l'alcane correspondant au groupe R augmenté d'un atome de carbone, puis l'hydrogénolyse.

L'invention fournit aussi un procédé de préparation d'un diol fluoré selon l'invention, lorsque celui-ci est saturé, comprenant la réaction radicalaire de CₙF₂ₙ₊₁I sur le monoallyl éther de triméthylol alcane, l'alcane correspondant au groupe R augmenté d'un atome de carbone, puis la déshydroiodation, puis l'hydrogénation.

Le diol fluoré selon l'invention est en particulier exempt de soufre.

Les diisocyanates et les composés à insaturation éthylènique tels que éthers vinyliques et acrylates sont les composés classiquement utilisés dans le domaine considéré. Ces composés peuvent être fluorés ou non. Le diisocyanate pourrait être remplacé par un polyisocyanate, mais à des fins de commodité c'est le premier terme qui est retenu comme terme générique. Des exemples de tels composés diisocyanates, acrylates et éthers vinyliques peuvent être trouvés par exemple parmi les composés cités dans la demande EP-A-0565425 et la demande FR-A-2712291.

Pour la préparation des revêtements de fibres, le matériau selon l'invention, qui présente entre autres la particularité d'être réticulable, est photoréticulé, en général par U.V., de préférence en présence d'un diluant réactif qui est en général un diacrylate, présent en une quantité classique.

On utilise classiquement des photoinitiateurs et/ou des catalyseurs pour les réactions (photo)chimiques, si besoin est.

Les diols fluorés de l'invention, dans lesquels R est C₂H₅, sont préparés par réaction radicalaire de CₙF₂ₙ₊₁I sur l'allyloxy triméthylol propane (ou monoallyléther de triméthylol propane), suivie soit d'une déshydroiodation éventuellement suivie d'une hydrogénation, soit d'une réduction directe ou d'une hydrogénolyse.

La description étant donnée en référence au diol dans lequel R est C₂H₅, il est clair que les autres composés sont préparés de la même façon, en partant du monoallyléther approprié. Ce monoallyl éther est le monoallyl éther de triméthylol alcane, l'alcane correspondant au groupe R augmenté d'un atome de carbone.

L'addition radicalaire peut être effectuée selon des modes opératoires bien connus, soit en masse, soit dans un solvant organique, soit dans l'eau.

Une telle addition radicalaire est décrite dans la demande de brevet DE-A-2 336 913, dont les conditions réactionnelles pourront être suivies.

Comme solvant organique, on peut utiliser l'acétone, le tétrahydrofurane, le dioxanne, le diméthylformamide, la N-méthyl-pyrrolidone-2, le diméthylsulfoxyde, la méthyl éthyl cétone, la méthyl isobutyl cétone, l'éthanol, l'isopropanol et l'acétate d'isopropyle. On utilisera de préférence un solvant ou un mélange de solvants hydrosoluble(s).

On effectue en général l'addition radicalaire en présence d'amorceur(s) qu'on utilise à raison de 0,1 à 1,5 % par rapport au poids total des monomères engagés, de préférence 0.1 à 0.5 %. Comme amorceurs, on peut utiliser des peroxydes tels que, par exemple, le peroxyde de benzoyle, le peroxyde de lauroyle, le peroxyde de succinyle et le perpivalate de tertiobutyle, ou des composés azoïques tels que l'azo-2,2'-bis-isobutyronitrile, l'azo-4,4'-bis-(cyano-4-pentanoïque) et l'azo-2,2'-bis-2-méthyl butanenitrile.

La température de réaction peut varier dans de larges limites, c'est-à-dire entre la température ambiante et le point d'ébullition du mélange de réaction. Préférentiellement, on opère entre 60 et 90°C; (on évite ainsi la formation de polymères). Dans le même but, on peut procéder par coulée de l'allyloxy triméthylol propane, ce qui permet de contrôler la réaction et de limiter l'élévation de température.

On obtient ainsi un produit d'addition iodé.

La déshydroiodation est effectuée à l'aide d'une base forte inorganique telle que la soude ou la potasse, ou une base forte organique telle que le DBU (1,8-diazabicyclo(5.4.0)undec-7en). La réaction est mise en oeuvre de préférence en milieu aqueux. La quantité de base forte utilisée est par exemple voisine de la stoechiométrie. La température est en général limitée aux environs de 70-75°C (on évite ainsi la formation de polymères).

On obtient ainsi un diol insaturé.

L'obtention des diols saturés peut être réalisée selon différentes méthodes. Ils peuvent être obtenus à partir du produit d'addition iodé par hydrogénolyse en présence d'un agent alcalin, ou bien par réduction avec le borbhydrure de sodium ou de zinc ou l'hydrure d'aluminium lithium ou l'hydrure de tributyl étain. Ils peuvent aussi être obtenus à partir du dérivé insaturé par hydrogénation catalytique selon des méthodes connues, sans solvant ou bien en solution dans un solvant organique classique tel que l'éthanol ou le méthanol, en présence d'un catalyseur d'hydrogénation qui peut être, selon les cas, soit du nickel Raney, soit du palladium sur charbon.

On obtient ainsi un diol saturé.

Le groupe perfluoroalkyle CₙF₂ₙ₊₁ peut être linéaire ou ramifié. Le composé CₙF₂ₙ₊₁I est connu en soi et est préparé par des procédés classiques.

Les exemples suivants illustrent l'invention sans la limiter.

### Exemple 1

a) CₙF₂ₙ₊₁-CH₂CHI-CH₂OCH₂-C(CH₂OH)₂ C₂H₅ : Dans un réacteur d'un litre, chauffé par une double enveloppe thermostatée, muni d'une agitation ancre et d'un réfrigérant à reflux, on introduit 125 grammes en poids d'un mélange d'iodures de perfluoroalkyles de formule : CₙF₂ₙ₊₁I où n est égal à 6,8,10,12 et 14 dans des rapports respectifs en poids de 63:25:8:2:2 (0.25 mole), en solution dans 200 grammes d'acétone, et 64.5 grammes de triméthylolpropane monoallyléther (0.375 mole)
   Après inertage à l'azote et chauffage à 62°C, on initie l'addition radicalaire par 0.4 gramme d'azo-bis-isobutyronitrile (AIBN), puis on rajoute 0.2 gramme d'AIBN toutes les 2 heures. La conversion de l'iodure de perfluoroalkyle CₙF₂ₙ₊₁I est suivie par chromatographie en phase gaz. La réaction est complète au bout de 20 heures. Après évaporation de l'acétone sous pression réduite, la masse réactionnelle est lavée trois fois par 200 grammes d'eau déminéralisée à 60°C pour éliminer l'excès de triméthylolpropane monoallyléther. Le produit d'addition, sous forme d'un liquide visqueux jaune clair, est séparé par décantation, puis séché sous vide. Il cristallise lentement à température ambiante, son point de fusion est d'environ 41°C. Le rendement par rapport au CₙF₂ₙ₊₁I est pratiquement quantitatif.
b) CₙF₂ₙ₊₁-CH=CH-CH₂OCH₂-C(CH₂OH)₂-C₂H₅ : Dans le même réacteur que ci-dessus, on procède à la même réaction, sur des quantités identiques. Après décantation, la phase aqueuse de lavage est retirée par aspiration, puis le produit d'addition obtenu est déshydro-iodé sans être isolé. Pour cela, on le maintient à 50°C et on coule goutte à goutte une solution aqueuse de soude soit 10.7 grammes de soude (0.27mole) dans 50 grammes d'eau, en une heure, de façon à ne pas dépasser 65°C. La conversion du dérivé iodé est suivie par chromatographie en phase gaz ; elle est totale après 7 heures. Après décantation, on élimine la phase aqueuse par aspiration, puis on lave la phase organique à l'eau déminéralisée à 50°C jusqu'à neutralité. Après séparation de la phase aqueuse, le diol fluoré obtenu est séché par distillation azéotropique avec du cyclohexane. Le solvant est ensuite distillé sous pression réduite. On obtient alors un liquide visqueux jaune clair avec un rendement de 95 % par rapport au CₙF₂ₙ₊₁I de départ.

### Exemple 2

On procède comme à l'exemple 1, en remplaçant le mélange de CₙF₂ₙ₊₁I par C₆F₁₃-I. Les conditions expérimentales sont les mêmes et l'on obtient de façon quantitative le dérivé iodé d'addition, sous forme d'un solide jaune pâle qui fond à 46°C.

La structure a été confirmée par RMN du proton (300 MHz, CDCl3). Les δ sont les suivants :
4.42 ppm (-CH-I, quintuplet,1H),
3.60-3.80 ppm (-CH₂-OH massif complexe, 4H),
3.54 ppm (-OCH₂-C, singulet, 2H),
2.60-3.05 ppm (-CH₂CF₂-, massif complexe, 2H),
1.35 ppm (CH₂CH₃ quadruplet, 2H),
0.86 ppm (CH₂CH₃, triplet, 3H).

Après déshydro-iodation dans les mêmes conditions qu'à l'exemple 1-b), on obtient un liquide visqueux (v ≅ 1300 cps) jaune clair, de densité 1.43. Il s'agit d'un mélange contenant 1.3 % molaire de diol de départ, 73 % molaire de l'isomère trans, 25 % molaire de l'isomère cis du diol fluoré insaturé. L'analyse par RMN du proton (400 MHz, CDCl3) donne les signaux suivants :
Isomère trans :
   6.45 ppm (CF₂-CH_{A}=CH_{B},_{,}D*T*t, ³3_{HA-F}=4.2Hz, ³JH_{A}-H_{B}=15.8Hz, 1H),
   5.90 ppm (CH_{A}=CH_{B}-CH₂-O, D*T*t, ⁴J_{HB-F}=12Hz, 1H),
   4.15 ppm (=CH-CH₂O, massif complexe, 2H),
   3.55-3.75 ppm (CH₂OH, massif complexe, 4H),
   3.50 ppm (O-CH₂-C, singulet, 2H),
   1.34 ppm (-CH₂-CH₃, quadruplet, 2H),
   0.85 ppm (-CH₂-CH₃, triplet, 3H).
Isomère cis :
   6.25 ppm (CF₂-CH_{A}=CH_{B'},D*T*t, ³J_{HA-F}=2.5Hz, ³JH_{A}-H_{B}=12.5Hz, 1H),
   5.60 ppm (CH_{A}=CH_{B}-CH₂-O, D*T*t, ⁴J_{HB-F}=15.5Hz, 1H),
   4.28 ppm (=CH-CH₂O, massif complexe, 2H),
   3.55-3.75 ppm (CH₂OH, massif complexe, 4H),
   3.44 ppm (O-CH₂-C, singulet, 2H),
   1.34 ppm (-CH₂-CH₃, quadruplet, 2H),
   0.85 ppm (-CH₂-CH₃, triplet, 3H).

### Exemple 3

a) Dans un réacteur de 1litre, chauffé par une double enveloppe thermostatée, muni d'une agitation ancre et d'un réfrigérant à reflux, on introduit : 273 grammes d'iodure de perfluoroalkyle C₈F₁₇-I (0.5 mole), préalablement lavé à température ambiante par une solution aqueuse de bisulfite pour éliminer les traces d'impuretés iodées, 93 grammes de triméthylolpropane monoallyléther (0.53 mole), 120 grammes d'eau déminéralisée. Le mélange hétérogène est inerté à l'azote puis chauffé, sous bonne agitation, à 70°C. On ajoute alors 0.4 grammes d'amorceur AIBN. La réaction est exothermique et on refroidit la double enveloppe pendant quelques minutes pour ne pas dépasser 90°C. Puis on maintient la température à 75°C et on suit la conversion des réactifs par chromatographie en phase gaz. On rajoute 0.2 grammes d'AIBN toutes les deux heures. La conversion du C₈F₁₇-I est complète au bout de 6 heures. L'excès de diol de départ est éliminé par trois lavages de la phase organique à l'eau à 75°C. L'analyse de cette dernière confirme la présence du produit d'addition attendu comme composé majoritaire. Cet intermédiaire se solidifie rapidement (72°C).
b) Dans le même réacteur, on enchaîne la réaction de déshydroiodation, directement sur la phase organique A cette dernière maintenue à 70°C, on ajoute 60 grammes d'eau à 60°C, puis on coule goutte à goutte en une heure, de façon à ne pas dépasser 68°C, une solution contenant 20.3 grammes de soude (0.5 mole) dans 80 grammes d'eau. Au bout de 6 heures, l'analyse par GC montre que la réaction est terminée. Après décantation, on élimine la phase aqueuse par aspiration, puis on lave la phase organique à l'eau déminéralisée à 50°C jusqu'à neutralité. Après séparation de la phase aqueuse, le diol fluoré obtenu est séché par distilïation azéotropique avec du cyclohexane. Le solvant est ensuite distillé sous pression réduite. On obtient alors un liquide visqueux jaune clair, qui se solidifie lentement à température ambiante (P_{fusion} = 38°C), avec un rendement de 95 % par rapport au C₈F₁₇-I de départ. Il s'agit d'un mélange contenant 0.7 % molaire de diol de départ, 73.1 % molaire de l'isomère trans, 24.5 % molaire de l'isomère cis. L'analyse par RMN du carbone 13 (75.5 MHz, CDCl3) donne :
   - pour l'isomère trans les signaux suivants :
      138.8 ppm (CF₂-CH=CH-, ³J_{C-F}=9Hz),
      117.3 ppm (CF₂-CH=CH, ²J_{C-F}=23.7Hz),
      73.4 ppm (CH₂O-CH₂-C-),
      69.6 ppm (=CH-CH₂O),
      66.1 ppm (-CH₂OH),
      43 ppm (C(CH₂OH)₂),
      23.0 ppm (CH₃-CH₂-),
      7.4 ppm (CH₃-CH₂-).
   - pour l'isomère cis les signaux suivants :
      142.3 ppm (CF₂-CH=CH-, ³J_{C-F}=5.6Hz),
      117 ppm (CF₂-CH=CH, ²J_{C-F}=23.7Hz),
      73.8 ppm (CH₂O-CH₂-C-),
      67.5 ppm (=CH-CH₂O),
      66.0 ppm (-CH₂OH),
      42.9 ppm (C(CH₂OH)₂),
      23.0 ppm (CH₃-CH₂-),
      7.4 ppm (CH₃-CH₂-).

### Exemple 4 C₆F₁₅-(CH₂)₃OCH₂-C(CH₂OH)₂ C₂H₅

Dans un autoclave agité d'une contenance d'environ 1 litre, on introduit 492 grammes (1mole) de diol fluoré en C₆F₁₃- décrit à l'exemple 2 dans 370 grammes de méthanol, et 20 grammes de palladium sur carbone à 5% et 10 grammes de K₂CO₃. On charge l'hydrogène jusqu'à une pression de 20 bars. La réaction est exothermique; la température monte à 50°C tandis que la pression tombe à 5 bars en 10 mn. On recharge en hydrogène à une pression de 20 bars jusqu'à ce qu'il n'y ait plus d'absorption d'hydrogène. On termine alors la réaction à 40°C sous 60 bars. L'analyse par chromatographie en phase-gaz montre que l'on a transformé la totalité du diol insaturé On sépare le catalyseur par filtration, puis on élimine le solvant sous pression réduite. La structure du diol saturé, obtenu avec un rendement de 97%, est confirmée par RMN du proton (400 MHz, CDCl3) :

On observe:
- la disparition des signaux à 6.45 et 6.25 ppm (CF₂-CH_{A}=CH_{B}) des isomères cis/trans
- la disparition des signaux à 5.90 et 5.60 ppm (CH_{A}=CH_{B}-CH₂-O) des isomères cis/trans
   3.5 ppm (-CH₂-CH₂O, triplet),
   3.6-3.7 ppm (CH₂OH, massif complexe, 4H),
   3.44 ppm (O-CH₂-C, singulet, 2H),
   2.15 ppm (CF₂-CH₂-, multiplet large),
   1.89 ppm (CF₂-CH₂-CH₂-, multiplet large),
   1.34 ppm (-CH₂-CH₃, quadruplet, 2H),
   0.85 ppm (-CH₂-CH₃, triplet, 3H).

### Exemple 5

Dans un autoclave agité d'une contenance d'environ 0.5 litre, on introduit 62 grammes (0.1mole) de produit iodé d'addition en C₆F₁₃- décrit à l'exemple 2, 13.8 grammes (0.11mole) de carbonate de potassium en poudre fine, 160 grammes d'éthanol absolu, 6 grammes de catalyseur palladium sur carbone 5 %. Après avoir purgé à l'azote, on charge l'hydrogène jusqu'à une pression de 50 bars.et on chauffe à 60°C sous agitation. La pression se stabilise aux environs de 40 bars au bout de 20 heures. On sépare le catalyseur et le carbonate par filtration, puis on élimine le solvant sous pression réduite. Le liquide visqueux obtenu est redissous dans 200 ml de chlorure de méthylène. La phase organique est lavée à l'eau pour éliminer le KI, séchée sur sulfate de sodium. Après filtration, puis élimination du solvant, le liquide visqueux obtenu, qui se solidifie rapidement, ne contient plus de produit iodé d'addition (analyse par chromatographie en phase gaz). On obtient 47g (soit un rendement de 95 %), du même produit qu'à l'exemple 4.

### Exemple 6

On procède comme à l'exemple 4, en remplaçant le diol en C₆F₁₃- par celui en C₈F₁₇- obtenu à l'exemple 3. Dans des conditions identiques, on obtient le diol fluoré saturé solide avec un rendement de 96 %.

### Exemple 7

Dans un réacteur de 5 litres, on introduit 610 g de triméthyl-2,2,4-hexaméthylène diisocyanate et 26,5 g de dibutyl dilaurate d'étain. On ajoute 715 g du diol fluoré de l'exemple 2, et on laisse réagir le mélange 1 heure à 80°C. On laisse redescendre la température à 40°C, puis on ajoute 2,9 g d'ionol, 492,7 g d'hexaméthylène diacrylate, et 343,8 g de 2-hydroxyéthylacrylate. On laisse la réaction se poursuivre jusqu'à observer par analyse infra-rouge la disparition de la bande isocyanate à 2260 cm⁻¹. On ajoute alors 5% en poids de photoamorceur Irgacure 184.

On procède ensuite au revêtement d'un fibre, dont la couche primaire est une couche standard et la couche secondaire est une couche en le matériau obtenu précédemment. La photopolymérisation est mise en oeuvre sous U.V.

On procède ensuite à la mesure de fatigue statique par la méthode avec deux points de flexion. Des longueurs de 2,5 cm de fibres étant lovées dans des tubes de précision en verre ; 20 fibres sont placées par tube. 5 tubes de diamètre différents sont utilisés, imposant les contraintes suivantes aux fibres :
1. 456 kpsi (3144 MPa)
2. 419 kpsi (2889 MPa)
3. 399 kpsi (2751 MPa)
4. 386 kpsi (2661 MPa)
5. 353 kpsi (2434 MPa)

Les fibres sous contraintes sont placées dans une enceinte climatique à 85°C, 85% d'humidité relative. Un détecteur acoustique déclenche le relevé automatique des temps de rupture de chacune des fibres. Les temps de rupture en fonction des contraintes appliquées sont tracés en échelle logarithmique et le facteur de fatigue statique n est calculé à partir de la pente de la droite obtenue. Dans le cas du revêtement standard on obtient une valeur de n de 18-20, tandis qu'avec la composition à base du diol fluoré selon l'invention, la valeur de n est de 23.

L'invention n'est pas limitée aux modes de réalisation décrits; le diol fluoré peut trouver d'autres applications, telles que l'utilisation comme agent modificateur de surface, notamment comme agent oléophobe et/ou hydrophobe.

## Revendications

1. Diol fluoré répondant à la formule (I):
CₙF₂ₙ₊₁―A―CH₂OCH₂―C(CH₂OH)₂―R
dans laquelle n vaut de 2 à 20 et A signifie ―CH=CH― ou ―CH₂CH₂― et R est un groupe alkyle contenant 1 à 4 atomes de carbone.

2. Diol fluoré selon la revendication 1, qui est insaturé et correspond à la formule:
CₙF₂ₙ₊₁―CH=CH―CH₂OCH₂―C(CH₂OH)₂―R.

3. Diol fluoré selon la revendication 1, qui est saturé et correspond à la formule:
CₙF₂ₙ₊₁―CH₂CH₂―CH₂OCH₂―C(CH₂OH)₂―R.

4. Diol fluoré selon la revendication 1, 2 ou 3, dans lequel R est C₂H₅.

5. Diol fluoré selon l'une quelconque des revendications 1 à 4, dans lequel dans la formule (I), n est un entier et est compris entre 6 et 14, inclusivement.

6. Diol fluoré selon l'une quelconque des revendications 1 à 4, dans lequel dans la formule (I), CₙF₂ₙ₊₁ résulte d'un mélange et n est compris entre 6 et 14, inclusivement.

7. Diol fluoré selon l'une quelconque des revendications 1 à 6, dans lequel dans la formule (I), n est compris entre 6 et 8, inclusivement.

8. Procédé de préparation d'un diol fluoré selon l'une quelconque des revendications 1, 2, 4, 5, 6 ou 7, comprenant la réaction radicalaire de CₙF₂ₙ₊₁I sur le monoallyl éther de triméthylol alcane, l'alcane correspondant au groupe R augmenté d'un atome de carbone, puis la déshydroiodation.

9. Procédé de préparation d'un diol fluoré selon l'une quelconque des revendications 1, 3, 4, 5, 6 ou 7, comprenant la réaction radicalaire de CₙF₂ₙ₊₁I sur le monoallyl éther de triméthylol alcane, l'alcane correspondant au groupe R augmenté d'un atome de carbone, puis la réduction directe.

10. Procédé de préparation d'un diol fluoré selon l'une quelconque des revendications 1, 3, 4, 5, 6 ou 7, comprenant la réaction radicalaire de CₙF₂ₙ₊₁I sur le monoallyl éther de triméthylol alcane, l'alcane correspondant au groupe R augmenté d'un atome de carbone, puis l'hydrogénolyse.

11. Procédé de préparation d'un diol fluoré selon l'une quelconque des revendications 1, 3, 4, 5, 6 ou 7, comprenant la réaction radicalaire de CₙF₂ₙ₊₁I sur le monoallyl éther de triméthylol alcane, l'alcane correspondant au groupe R augmenté d'un atome de carbone, puis la déshydroiodation, puis l'hydrogénation.

## Claims

1. A fluorinated diol of the formula (I):
CₙF₂ₙ₊₁-A-CH₂OCH₂-C(CH₂OH)₂-R
in which n has a value of 2 to 20 and A means -CH=CH- or -CH₂CH₂- and
R is an alkyl group containing 1 to 4 carbon atoms.

2. A fluorinated diol according to claim 1 which is unsaturated and corresponds to the formula:
CₙF₂ₙ₊₁-CH=CH-CH₂OCH₂-C(CH₂OH)₂-R.

3. A fluorinated diol according to claim 1 which is saturated and corresponds to the formula:
CₙF₂ₙ₊₁-CH₂CH₂-CH₂OCH₂-C(CH₂OH)₂-R.

4. A fluorinated diol according to claim 1, 2 or 3, in which R is C₂H₅.

5. A fluorinated diol according to any one of claims 1 to 4, in which, in the formula (I), n is an integer and is between 6 and 14 inclusive.

6. A fluorinated diol according to any one of claims 1 to 4, in which, in the formula (I), CₙF₂ₙ₊₁ is the result of a mixture and n is between 6 and 14 inclusive.

7. A fluorinated diol according to any one of claims 1 to 6, in which, in the formula (I), n is an integer and is between 6 and 8 inclusive.

8. A process for the preparation of a fluorinated diol according to any one of claims 1, 2, 4, 5, 6 or 7, comprising the free-radical reaction of CₙF₂ₙ₊₁I on the trimethylolalkane monoallyl ether, the alkane corresponding to the group R with one additional carbon atom, followed by dehydroiodation.

9. A process for the preparation of a fluorinated diol according to any one of claims 1, 3, 4, 5, 6 or 7, comprising the free-radical reaction of CₙF₂ₙ₊₁I on the trimethylolalkane monoallyl ether, the alkane corresponding to the group R with one additional carbon atom, followed by direct reduction.

10. A process for the preparation of a fluorinated diol according to any one of claims 1, 3, 4, 5, 6 or 7, comprising the free-radical reaction of CₙF₂ₙ₊₁I on the trimethylolalkane monoallyl ether, the alkane corresponding to the group R with one additional carbon atom, followed by hydrogenolysis.

11. A process for the preparation of a fluorinated diol according to any one of claims 1, 3, 4, 5, 6 or 7, comprising the free-radical reaction of CₙF₂ₙ₊₁I on the trimethylolalkane monoallyl ether, the alkane corresponding to the group R with one additional carbon atom, followed by dehydroiodation, followed by hydrogenation.

## Patentansprüche

1. Fluoriertes Diol, das der folgenden Formel (I):
CₙF₂ₙ₊₁-A-CH₂OCH₂-C(CH₂OH)₂-R
entspricht, bei der n 2 bis 20 ist, und A -CH=CH- oder -CH₂CH₂- bedeutet, und R eine Alkylgruppe ist, die 1 bis 4 Kohlenstoffatome enthält.

2. Fluoriertes Diol gemäß Anspruch 1, das ungesättigt ist und der Formel:
CₙF₂ₙ₊₁-CH=CH-CH₂OCH₂-C(CH₂OH)₂-R
entspricht.

3. Fluoriertes Diol gemäß Anspruch 1, das gesättigt ist und der Formel:
CₙF₂ₙ₊₁-CH₂CH₂-CH₂OCH₂-C(CH₂OH)₂-R
entspricht.

4. Fluoriertes Diol gemäß Anspruch 1, 2 oder 3, bei dem R C₂H₅ ist.

5. Fluoriertes Diol gemäß irgendeinem der Ansprüche 1 bis 4, bei dem in der Formel (I) n eine ganze Zahl von 6 bis einschließlich 14 ist.

6. Fluoriertes Diol gemäß irgendeinem der Ansprüche 1 bis 4, bei dem in der Formel (I) CₙF₂ₙ₊₁ sich aus einem Gemisch ergibt, und n 6 bis einschließlich 14 ist.

7. Fluoriertes Diol gemäß irgendeinem der Ansprüche 1 bis 6, bei dem in der Formel (I) n 6 bis einschließlich 8 ist.

8. Verfahren zur Herstellung eines fluorierten Diols gemäß irgendeinem der Ansprüche 1, 2, 4, 5, 6 oder 7, aufweisend die Radikalen-Reaktion CₙF₂ₙ₊₁I bei dem Trimethylolalkan-monoallylether, wobei das Alkan der um ein Kohlenstoffatom vergrößerten Gruppe R entspricht, und dann die Dehydroiodierung.

9. Verfahren zur Herstellung eines fluorierten Diols gemäß irgendeinem der Ansprüche 1, 3, 4, 5, 6 oder 7, aufweisend die Radikalen-Reaktion CₙF₂ₙ₊₁I bei dem Trimethylolalkan-monoallylether, wobei das Alkan der um ein Kohlenstoffatom vergrößerten Gruppe R entspricht, und dann die direkte Reduktion.

10. Verfahren zur Herstellung eines fluorierten Diols gemäß irgendeinem der Ansprüche 1, 3, 4, 5, 6 oder 7, aufweisend die Radikalen-Reaktion CₙF₂ₙ₊₁I bei dem Trimethylolalkan-monoallylether, wobei das Alkan der um ein Kohlenstoffatom vergrößerten Gruppe R entspricht, und dann die Hydrogenolyse.

11. Verfahren zur Herstellung eines fluorierten Diols gemäß irgendeinem der Ansprüche 1, 3, 4, 5, 6 oder 7, aufweisend die Radikalen-Reaktion CₙF₂ₙ₊₁I bei dem Trimethylolalkan-monoallylether, wobei das Alkan der um ein Kohlenstoffatom vergrößerten Gruppe R entspricht, und dann die Dehydroiodierung, und dann die Hydrierung.
